# EUROPÄISCHE PATENTANMELDUNG

(11) **EP 3 633 217 A1**
(43) Veröffentlichungstag der Anmeldung: **08.04.2020**
(21) Anmeldenummer: 18198373.5
(22) Anmeldetag: 02.10.2018
(51) Int. Cl.: F16C 23/04, F16C 17/08, F16C 35/02, F16C 33/10, A61M 1/00, A61M 1/10, A61M 1/12

(54) **LAGERBAUGRUPPE UND ROTATIONSFLUIDPUMPE**

(71) Anmelder: Berlin Heart GmbH, 12247 Berlin (DE)
(72) Erfinder: PIELKA, Sascha, 13158 Berlin (DE); SCHMIDT, Bodo, 14513 Teltow (DE)
(74) Vertreter: Pfenning, Meinig & Partner mbB

(57) **Zusammenfassung**

Die vorliegende Erfindung betrifft eine Lagerbaugruppe (1) zum Lagern eines um eine Rotationsachse (6) rotierbaren Rotors, insbesondere für eine Rotationsfluidpumpe oder Rotationsblutpumpe, umfassend: einen Grundkörper (2), ein relativ zum Grundkörper in Richtung der Rotationsachse verschiebbares Lagerelement (4) zum Aufnehmen des Rotors, und eine mit dem Lagerelement verbundene Stelleinrichtung (7, 8, 9) zum Verschieben des Lagerelements in der Richtung der Rotationsachse um einen vorbestimmten Weg, wobei der vorbestimmte Weg ≤ 500 µm beträgt.

## Beschreibung

Die vorliegende Erfindung betrifft eine Lagerbaugruppe zum Lagern eines um eine Rotationsachse rotierbaren Rotors, insbesondere für eine Rotationsfluidpumpe. Weiterhin betrifft die Erfindung eine Rotationsfluidpumpe zum Fördern eines Fluids, insbesondere eine Rotationsblutpumpe zum Fördern von Blut.

Im Stand der Technik sind Rotationsblutpumpen bekannt, bei denen der Rotor axial und radial rein mechanisch, mechanisch-magnetisch oder hydrodynamisch gelagert ist. Diese Lager weisen im Allgemeinen einen definierten Lagerspalt zwischen dem Rotor und dem Lagerelement des Stators auf, welches den Rotor im Bereich der Rotationsachse aufnimmt. Der Lagerspalt muss dabei einerseits so dimensioniert sein, dass zwischen die beiden Lagerkomponenten ein bestimmter Flüssigkeitsanteil des durch die Rotationsblutpumpe gepumpten Blutes zur Schmierung des Lagers gelangt. Die Schmierung hat dabei den Vorteil, dass die Reibung im Lager verringert wird, wodurch ein übermäßiges Erwärmen von im Blut enthaltenem Bluteiweiß verhindert wird. Andererseits sollen keine sich im Blut befindlichen festen Bestandteile in den Lagerspalt eindringen, da diese die Hämolyse erhöhen und das Thrombenwachstum fördern. Die Einstellung eines definierten Lagerspaltes ist somit für einen ruhigen Lauf des Rotors notwendig und hat einen sehr großen Einfluss auf das Blutschädigungspotential der Rotationsblutpumpe.

Bei bekannten mechanisch gelagerten Axialflusspumpen erfolgt die Einstellung des Lagerspaltes der beiden sich einlassseitig und auslassseitig befindlichen Ball-Cap-Lager durch aufwendiges Vermessen der Einzelkomponenten und anschließendem Anpassen der Teile durch manuelle Nacharbeit (z.B. durch Läppen, Schleifen, usw.). Dieses Verfahren ist jedoch manuell sehr aufwendig und nur begrenzt reproduzierbar. Trotz des hohen Arbeitsaufwands zur Einstellung eines definierten Lagerspaltes zeigen diese Axialflusspumpen ein hohes Thrombogenitätsrisiko.

Die Aufgabe der vorliegenden Erfindung ist es daher, eine Lagerbaugruppe bereitzustellen, welche es ermöglicht einen maßlich definierten Lagerspalt in einer Rotationsfluidpumpe reproduzierbar einzustellen. Weiterhin ist es eine Aufgabe der Erfindung eine Rotationsfluidpumpe zum Fördern eines Fluids mit einer erfindungsgemäßen Lagerbaugruppe zur Verfügung zu stellen.

Die Aufgabe wird durch eine erfindungsgemäße Lagerbaugruppe nach Anspruch 1 sowie eine Rotationsfluidpumpe nach Anspruch 15 gelöst. Vorteilhafte Weiterbildungen der erfindungsgemäßen Lagerbaugruppe sind in den abhängigen Ansprüchen aufgeführt.

Die erfindungsgemäße Lagerbaugruppe zum Lagern eines um eine Rotationsachse rotierbaren Rotors, insbesondere für eine Rotationsfluidpumpe, umfasst einen Grundkörper, ein relativ zum Grundkörper in Richtung der Rotationsachse verschiebbares Lagerelement zum Aufnehmen des Rotors, und eine mit dem Lagerelement verbundene Stelleinrichtung zum Verschieben des Lagerelements in der Richtung der Rotationsachse um einen vorbestimmten Weg, wobei der vorbestimmte Weg ≤ 500 µm beträgt.

Die erfindungsgemäße Lagerbaugruppe ermöglicht somit nach Fertigstellung und Montage der Lagerkomponenten zu einer Lagerbaugruppe und beispielsweise nach Einbau in eine Rotationsfluidpumpe die Position des Lagerelements im Mikrometerbereich zu verändern und einzustellen. Insbesondere ermöglicht die erfindungsgemäße Lagerbaugruppe den Lagerspalt zwischen dem Rotor und dem Lagerelement maßlich definiert und reproduzierbar einzustellen.

Die erfindungsgemäße Lagerbaugruppe kann beispielsweise in einer Rotationsblutpumpe zum Fördern von Blut, insbesondere in einer implantierbaren Rotationsblutpumpe, eingesetzt werden. In der Rotationsblutpumpe ermöglicht die erfindungsgemäße Lagerbaugruppe durch die maßlich definierte Einstellbarkeit des Lagerspalts zwischen dem Lagerelement und dem Rotor eine Verringerung des Blutschädigungspotenzials der Rotationsblutpumpe.

In einer besonders bevorzugten Ausgestaltungsvariante der erfindungsgemäßen Lagerbaugruppe kann der vorbestimmte Weg ≤ 250 µm, insbesondere ≤ 100 µm, insbesondere ≤ 50 µm, insbesondere ≤ 20 µm, bevorzugt ≤ 15 µm, besonders bevorzugt ≤ 10 µm, insbesondere ≤ 5 µm betragen. In diesen Fällen kann das Lagerelement besonders fein verschoben und der Lagerspalt besonders fein eingestellt werden.

Die Lagerbaugruppe kann Bestandteil eines mechanischen und/oder hydrodynamischen Lagers, insbesondere eines Ball-Cap-Lagers, eines mechanischmagnetischen Lagers und/oder eines Sicherheitslagers sein.

Das Lagerelement kann eine Lagerfläche zur Aufnahme des Rotors aufweisen, wobei die Stelleinrichtung auf einer der Lagerfläche abgewandten Seite des Lagerelements angeordnet ist. Bei Verwendung der Lagerbaugruppe in einem Pumpengehäuse befindet sich die Stelleinrichtung somit im Wesentlichen vorzugsweise außerhalb des eigentlichen Pumpengehäuses, damit diese von außen, auch nach Einbau der Lagerbaugruppe, bedienbar ist.

In einem weiteren bevorzugten Ausführungsbeispiel der Erfindung kann die Stelleinrichtung ein Schraubelement umfassen, welches derart mit dem Lagerelement verbunden ist, dass eine Drehung des Schraubelements um einen vorbestimmten Winkel eine Verschiebung des Lagerelements um den vorbestimmten Weg bewirkt.

Insbesondere kann eine Drehung des Schraubelements um 45 ° eine Verschiebung des Lagerelements um 25 µm bewirken. Dies ermöglicht eine sehr feine und in hohem Maße reproduzierbare Verschiebung des Lagerelements und somit eine sehr feine und hoch reproduzierbare Einstellung des Lagerspalts zwischen Rotor und Lagerelement.

Ein weiteres mögliches Ausführungsbeispiel sieht vor, dass die Stelleinrichtung eine Feingewindespindel und eine Gewindescheibe umfasst, wobei die Gewindescheibe mit dem Grundelement starr verbunden ist, und wobei die Feingewindespindel in der Richtung der Rotationsachse mit dem Lagerelement starr verbunden und in die Gewindescheibe eingeschraubt ist, sodass eine Schraubbewegung der Feingewindespindel in der Gewindescheibe um einen vorbestimmten Winkel eine Verschiebung des Lagerelements um den vorbestimmten Weg bewirkt. Nach Einstellung einer Position des Lagerelements kann die Feingewindespindel mit einer Kontermutter fixiert werden.

Besonders vorteilhaft ist es, wenn zwischen dem Lagerelement und der Gewindescheibe ein Federelement angeordnet ist, welches die Feingewindespindel gegenüber der Gewindescheibe vorspannt. Hierdurch kann ein Gewindespiel zwischen Gewindescheibe und Feingewindespindel reduziert werden.

Ein weiteres mögliches Ausführungsbeispiel sieht vor, dass die Stelleinrichtung eine Feingewindespindel, eine Segmentplatte mit mindestens vier kreuzförmig angeordneten Segmenten und eine Kontermutter aufweist, wobei die Feingewindespindel in der Richtung der Rotationsachse starr mit dem Lagerelement verbunden ist und auf einer dem Lagerelement abgewandten Seite in die Segmentplatte eingeschraubt und mittels der Kontermutter in der Segmentplatte fixiert ist, und wobei die Segmentplatte in zwei gegenüberliegenden Segmenten zum Grundkörper hin erhöhte Bereiche mit einer vorbestimmten Höhe aufweist, und der Grundkörper den erhöhten Bereichen der Segmentplatte gegenüberliegende vertiefte Bereiche aufweist, sodass in einer ersten Position der Segmentplatte die erhöhten und vertieften Bereiche ineinander greifen können, und sodass ein Drehen der Segmentplatte in eine zweite Position, in welcher die erhöhten und vertieften Bereiche nicht ineinander greifen, eine Verschiebung des Lagerelements um den vorbestimmten Weg bewirkt, wobei der vorbestimmte Weg der vorbestimmten Höhe der erhöhten Bereiche entspricht. Die erhöhten Bereiche können dabei insbesondere eine Höhe von ≥ 3 µm und/oder ≤ 20 µm, bevorzugt ≥ 5 µm und/oder ≤ 15 µm aufweisen.

Ein weiteres mögliches Ausführungsbeispiel sieht vor, dass die Stelleinrichtung eine Segmentplatte mit mindestens vier kreuzförmig angeordneten Segmenten und einer Klemmeinrichtung aufweist, wobei das Lagerelement in der Richtung der Rotationsachse in die Segmentplatte eingesteckt und mittels der Klemmeinrichtung fixiert ist, und wobei die Segmentplatte in zwei gegenüberliegenden Segmenten zum Grundkörper hin erhöhte Bereiche mit einer vorbestimmten Höhe aufweist, und der Grundkörper den erhöhten Bereichen der Segmentplatte gegenüberliegende vertiefte Bereiche aufweist, sodass in einer ersten Position der Segmentplatte die erhöhten und vertieften Bereiche ineinandergreifen können, und sodass ein Drehen der Segmentplatte in eine zweite Position, in welcher die erhöhten und vertieften Bereiche nicht ineinander greifen, eine Verschiebung des Lagerelements um den vorbestimmten Weg bewirkt, wobei der vorbestimmte Weg der vorbestimmten Höhe der erhöhten Bereiche entspricht. Die erhöhten Bereiche können dabei insbesondere eine Höhe von ≥ 3 µm und/oder ≤ 20 µm, bevorzugt ≥ 5 µm und/oder ≤ 15 µm aufweisen.

Insbesondere kann das Lagerelement in der Richtung der Rotationsachse mittels einer an ein Segment der Segmentplatte angreifenden und am Grundkörper befestigten Halteeinrichtung fixierbar sein.

Die erhöhten Bereiche können mittels eines Spanprozesses, insbesondere mittels eines Schleifprozesses, und/oder mittels eines additiven Prozesses, insbesondere mittels eines Aufdampfprozesses, eines Druckprozesses und/oder eines Beschichtungsprozesses, herstellbar sein.

Ein weiteres mögliches Ausführungsbeispiel sieht vor, dass die Stelleinrichtung eine Keilscheibe mit zwei diametral angeordneten, in einer Umfangsrichtung der Keilscheibe geneigten Keilflächen umfasst, wobei das Lagerelement in die Keilscheibe eingeschraubt ist, sodass ein Drehen der Keilscheibe um einen vorbestimmten Winkel eine Verschiebung des Lagerelements um den vorbestimmten Weg bewirkt.

Ein weiteres mögliches Ausführungsbeispiel sieht vor, dass das Lagerelement auf einer dem aufzunehmenden Rotor abgewandten Seite des Lagerelements einen keilförmigen Abschnitt aufweist, und die Stelleinrichtung ein scheibenförmiges Element mit einer trichterförmigen Durchgangsöffnung aufweist, wobei das Lagerelement mit dem keilförmigen Abschnitt in die trichterförmige Durchgangsöffnung eingesteckt ist und starr mit einem Abschlusselement verbunden ist, und wobei weiterhin die Stelleinrichtung ein Federelement, welches zwischen dem scheibenförmigen Element und dem Abschlusselement angeordnet ist und das scheibenförmige Element gegenüber dem Lagerelement vorspannt, und eine in dem scheibenförmigen Element angeordnete Klemmschraube umfasst, mit welcher ein Durchmesser der keilförmigen Durchgangsöffnung um einen vorbestimmten Betrag veränderbar ist, sodass ein Verändern des Durchmessers um einen vorbestimmten Differenzdurchmesser eine Verschiebung des Lagerelements um den vorbestimmten Weg bewirkt.

Ein weiteres mögliches Ausführungsbeispiel sieht vor, dass die Stelleinrichtung einen Hebel umfasst, der mit dem Lagerelement verbunden ist, sodass eine Veränderung der Neigung des Hebels relativ zum Grundkörper um einen vorbestimmten Differenzwinkel eine Verschiebung des Lagerelements um den vorbestimmten Weg bewirkt.

Ein weiteres mögliches Ausführungsbeispiel sieht vor, dass die Stelleinrichtung ein Element mit einem vorbestimmten Wärmeausdehnungskoeffizienten umfasst, wobei das Element derart mit dem Lagerelement verbunden ist, dass eine Änderung einer Temperatur des Elements um einen vorbestimmten Temperaturbetrag eine Verschiebung des Lagerelements um den vorbestimmten Weg bewirkt.

Ein weiteres mögliches Ausführungsbeispiel sieht vor, dass die Stelleinrichtung ein Piezo-Element umfasst, welches mit dem Lagerelement derart verbunden ist, dass ein Anlegen einer vorbestimmten elektrischen Spannung an das Piezo-Element ein Verschieben des Lagerelements um den vorbestimmten Weg bewirkt.

Insbesondere kann das Piezo-Element lösbar mit dem Lagerelement verbunden sein.

Die vorliegende Erfindung schließt auch eine Rotationsfluidpumpe zum Fördern eines Fluids, insbesondere eine Rotationsblutpumpe oder eine implantierbare Rotationsblutpumpe zum Fördern von Blut mit ein, die eine vorstehend beschriebene Lagerbaugruppe umfasst.

Insbesondere kann das Grundelement der Lagerbaugruppe ein Bestandteil eines Gehäusebodens eines Pumpengehäuses der Rotationsfluidpumpe sein, wobei sich die Stelleinrichtung insbesondere außerhalb des Pumpengehäuses befindet, um von außen, auch nach Einbau der Lagerbaugruppe in das Pumpengehäuse, bedienbar zu sein.

Im Folgenden werden mehrere Ausführungsbeispiele einer erfindungsgemäßen Lagerbaugruppe anhand von Figuren detaillierter beschrieben. Dabei werden verschiedene erfindungswesentliche oder auch vorteilhafte weiterbildende Elemente im Rahmen jeweils eines konkreten Beispiels genannt, wobei auch einzelne dieser Elemente als solche zur Weiterbildung der Erfindung - auch herausgelöst aus dem Kontext des Beispiels und weiterer Merkmale des Beispiels - verwendet werden können. Weiterhin werden in den Figuren für gleiche oder ähnliche Elemente gleiche oder ähnliche Bezugszeichen verwendet, und deren Erläuterung daher teilweise weggelassen.

Es zeigen
- Figur 1: eine Lagerbaugruppe gemäß einem ersten Ausführungsbeispiel der Erfindung in einer axialen Schnittansicht;
- Figur 2: eine Lagerbaugruppe gemäß einem zweiten Ausführungsbeispiel der Erfindung in einer axialen Schnittansicht;
- Figur 3: eine Lagerbaugruppe gemäß einem dritten Ausführungsbeispiel der Erfindung in einer axialen Schnittansicht;
- Figur 4: eine Lagerbaugruppe gemäß einem vierten Ausführungsbeispiel der Erfindung in einer axialen Schnittansicht;
- Figur 5: eine Keilscheibe als Bauteil einer Lagerbaugruppe gemäß einem fünften Ausführungsbeispiel der Erfindung in Perspektivansichten;
- Figur 6: eine Lagerbaugruppe gemäß einem sechsten Ausführungsbeispiel der Erfindung in einer axialen Schnittansicht;
- Figur 7: eine Lagerbaugruppe gemäß einem siebten Ausführungsbeispiel der Erfindung in einer axialen Schnittansicht;
- Figur 8: eine Lagerbaugruppe gemäß einem achten Ausführungsbeispiel der Erfindung in einer axialen Schnittansicht;
- Figur 9: eine Lagerbaugruppe gemäß einem neunten Ausführungsbeispiel der Erfindung in einer axialen Schnittansicht.

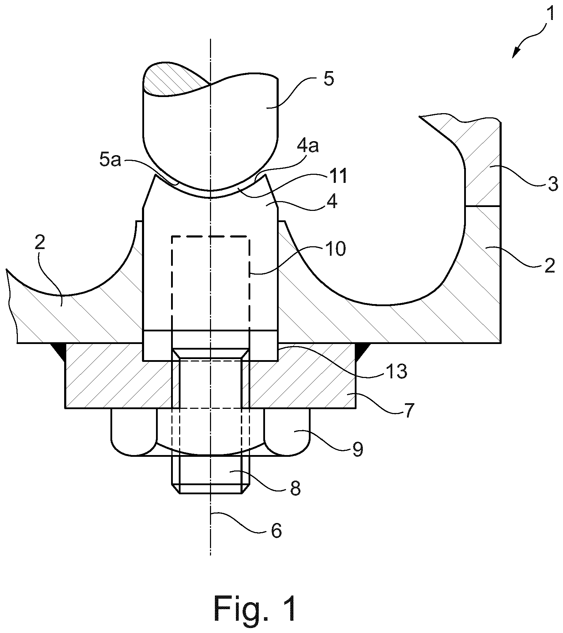

Figur 1 zeigt eine Lagerbaugruppe 1 und deren Einbau in eine Rotationsfluidpumpe gemäß einem ersten Ausführungsbeispiel der Erfindung in einer axialen Schnittansicht. Die Lagerbaugruppe 1 weist ein Grundelement 2 auf, welches als Gehäuseboden 2 der Rotationsfluidpumpe ausgebildet ist. Die Lagerbaugruppe 1 ist im Bereich eines Fluidauslasses der Rotationsfluidpumpe angeordnet. In Richtung eines stromaufwärts angeordneten Fluideinlasses schließt sich ein weiterer Gehäuseteil 3 an den Gehäuseboden 2 der Rotationsfluidpumpe an. Der Gehäuseboden 2 und der Gehäuseteil 3 bilden Teile des hier nicht vollständig dargestellten Pumpengehäuses. In etwa mittig durch den Gehäuseboden 2 verläuft senkrecht zur Haupterstreckungsebene des Gehäusebodens 2 ein erstes Lagerelement 4 der Lagerbaugruppe 1 und führt durch den Gehäuseboden 2 hindurch. Das erste Lagerelement 4 ist in einer senkrechten Richtung zur Haupterstreckungsebene des Gehäusebodens 2 verschieblich in dem Gehäuseboden 2 angeordnet. Auf dem Lagerelement 4 ist ein fest mit einem hier nicht dargestellten Rotor der Rotationsfluidpumpe verbundenes zweites Lagerelement 5 gelagert. Das erste Lagerelement 4 weist eine kalottenförmige Lagerfläche 4a auf, in welche eine sphärische Lagerfläche 5a des zweiten Lagerelements 5 eingreift. Bei dem hier dargestellten Lager der Rotationsfluidpumpe handelt es sich um ein Ball-Cap-Lager. Dieses ermöglicht, dass sich das zweite Lagerelement 5 in dem ersten Lagerelement 4 um eine im Wesentlichen senkrecht zur Haupterstreckungsebene des Gehäusebodens 2 und parallel zur Durchtrittsrichtung des Lagerelements 4 durch den Gehäuseboden 2 verlaufende Rotationsachse 6 drehen kann. Die Position des zweiten Lagerelements 5 entlang der Rotationsachse 6, also in axialer Richtung, ist im Wesentlichen durch ein einlassseitig angeordnetes, hier nicht dargestelltes zweites Pumpenlager festgelegt. Die Lagerbaugruppe 1 verfügt zur Einstellung einer axialen Position des ersten Lagerelements 4 und damit eines Lagerspalts 11 zwischen dem ersten Lagerelement 4 und dem zweiten Lagerelement 5 über eine Stelleinrichtung 7, 8, 9, welche auf einer der Lagerfläche 4a des ersten Lagerelements 4 gegenüberliegenden Seite des ersten Lagerelements 4 außerhalb des Pumpengehäuses angeordnet ist. Die Stelleinrichtung 7, 8, 9 weist eine starr mit dem Gehäuseboden 2 formschlüssig verbundene Gewindescheibe 7 auf, sowie eine starr mit dem ersten Lagerelement 4 verbundene Feingewindespindel 8 auf. Die Feingewindespindel 8 ist in eine zylinderförmige Öffnung 10 des ersten Lagerelements 4 koaxial eingeführt und innerhalb des ersten Lagerelements 4 mit dem ersten Lagerelement 4 verklebt oder verschweißt. Die Gewindescheibe 7 und die Feingewindespindel 8 weisen zueinander angepasste Gewinde auf, sodass die Feingewindespindel 8 in die Gewindescheibe 7 einschraubbar und aus dieser herausschraubbar ist. Dabei verlaufen Gewindeachsen der Feingewindespindel 8 und der Gewindescheibe 7 koaxial zur Rotationsachse 6. Durch Ein- und Ausschrauben der Gewindespindel 8 in die Gewindescheibe 7 oder aus der Gewindescheibe 7 heraus kann das erste Lagerelement 4 weiter durch den Gehäuseboden 2 in Richtung des Fluideinlasses in das Pumpengehäuse eingeschraubt werden, sodass sich der Lagerspalt 11 verkleinert, oder aus dem Pumpengehäuse herausgeschraubt werden, sodass sich der Lagerspalt 11 vergrößert. Die Gewindescheibe 7 weist auf einer dem ersten Lagerelement 4 zugewandten Seite eine Vertiefung 13 auf, deren Durchmesser an einen Durchmesser des Lagerelements 4 angepasst ist, sodass das Lagerelement 4, wenn dieses weit genug aus dem Pumpengehäuse herausgeschraubt wird, in die Vertiefung 13 formschlüssig eingreifen kann. Eine Drehung der Feingewindespindel 8 um 45° führt dabei etwa zu einer axialen Wegdifferenz des ersten Lagerelements 4 von 25 µm. Somit ist die axiale Position des ersten Lagerelements 4 sehr fein und reproduzierbar einstellbar. Um die axiale Position des ersten Lagerelements 4 festzuhalten, ist auf einer dem ersten Lagerelement 4 abgewandten Seite der Gewindescheibe 7 eine Kontermutter 9 auf die Feingewindespindel 8 aufgeschraubt.

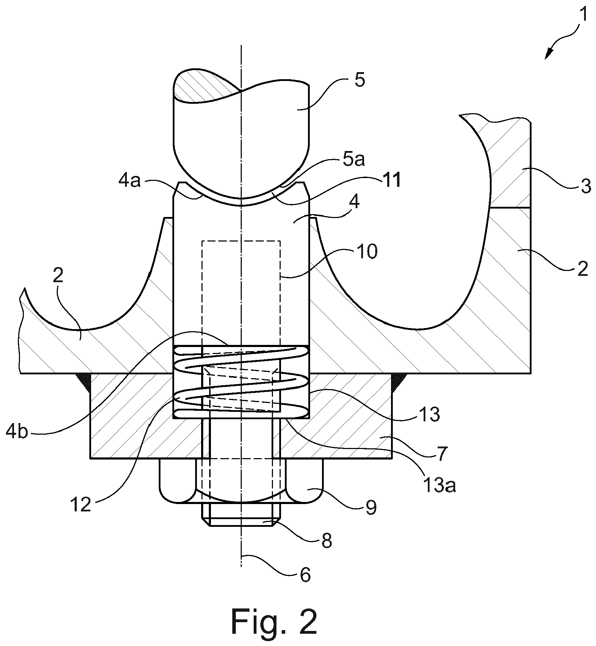

Figur 2 zeigt eine Lagerbaugruppe gemäß einem zweiten Ausführungsbeispiel der Erfindung sowie deren Einbau in eine Rotationsfluidpumpe in einer axialen Schnittansicht. Die Lagerbaugruppe 1 gemäß dem zweiten Ausführungsbeispiel sowie deren Einbau in eine Rotationsfluidpumpe ist ähnlich wie im ersten Ausführungsbeispiel. Im Unterschied zum ersten Ausführungsbeispiel ist die Vertiefung 13 der Gewindescheibe 7 in axialer Richtung verlängert ausgebildet. Weiterhin ist zwischen einer der Lagerfläche 4a gegenüberliegenden Fläche 4b des Lagerelements 4 und einer Bodenfläche 13a der Vertiefung 13 eine Spiralfeder 12 angeordnet, die die Feingewindespindel 8 und das erste Lagerelement 4 gegenüber der Gewindescheibe 7 vorspannt. Auf diese Weise kann ein Gewindespiel zwischen der Gewindescheibe 7 und der Feingewindespindel 8 reduziert werden und dadurch die axiale Position des ersten Lagerelements 4 noch stärker reproduzierbar eingestellt werden.

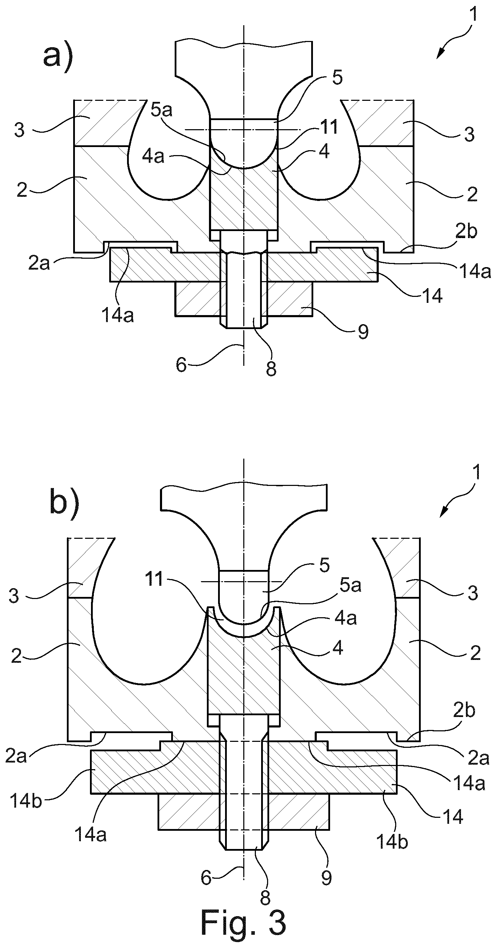

Figur 3 zeigt eine Lagerbaugruppe gemäß einem dritten Ausführungsbeispiel der Erfindung sowie deren Einbau in einer Rotationsfluidpumpe in einer axialen Schnittansicht. Die Lagerbaugruppe 1 des dritten Ausführungsbeispiels ist ähnlich wie die vorangegangenen Ausführungsbeispiele aufgebaut. Das dritte Ausführungsbeispiel unterscheidet sich lediglich durch die konkrete Ausbildung der Stelleinrichtung. Die Stelleinrichtung 14, 8, 9, 2a weist hier im Wesentlichen eine starr mit dem ersten Lagerelement 4 verbundene Feingewindespindel 8 wie in den Figuren 1 und 2, eine Segmentplatte 14 mit mindestens vier kreuzförmig angeordneten Segmenten 14a, 14b anstelle der Gewindescheibe 7 und eine Kontermutter 9 auf. Die Segmentplatte 14 ist formschlüssig auf einer der Lagerfläche 4a abgewandten Seite des Gehäusebodens 2 am Gehäuseboden 2 angeordnet. Die Feingewindespindel 8 ist in ähnlich wie in die Gewindescheibe 7 der Figuren 1 und 2 in die Segmentplatte 14 eingeschraubt und somit in axialer Richtung starr mit der Segmentplatte 14 verbunden. Zwei gegenüberliegende Segmente 14a der vier Segmente 14a, 14b weisen zum Gehäuseboden 2 hin erhöhte Bereiche mit einer vorbestimmten Höhe auf. Der Gehäuseboden 2 weist auf seiner der Lagerfläche 4a abgewandten Seite zu den erhöhten Bereichen der Segmente 14a korrespondierende vertiefte Bereiche 2a auf, sodass die erhöhten Bereiche in die vertieften Bereiche 2a eingreifen können (siehe Figur 3a)). Die weiteren Segmente 14b verlaufen in Figur 3a) bevorzugt senkrecht zur Zeichenebene, also auch senkrecht zu den Segmenten 14a. In der in Figur 3a) gezeigten Position der Segmentplatte 14, in der die erhöhten Bereiche der Segmente 14a vollständig in die vertieften Bereiche 2a eingreifen, liegen der Gehäuseboden 2 und die Segmente 14b formschlüssig aufeinander. In dieser Position der Stelleinrichtung liegen auch die Lagerflächen 4a und 5a der Lagerelemente 4 und 5 direkt aufeinander. Der Lagerspalt ist somit Null. In Figur 3b) liegen dagegen die Segmente 14b ohne erhöhte Bereiche in der Zeichenebene, also gegenüber den vertieften Bereichen 2a. Die Segmente 14a liegen im Wesentlichen senkrecht zu den Segmenten 14b, also verlaufen senkrecht zur Zeichenebene. Dabei befinden sich die erhöhten Bereiche der Segmente 14a nicht gegenüber von vertieften Bereichen 2a des Gehäusebodens 2 und können in diese nicht eingreifen, sondern liegen an der Gehäusebodenfläche 2b an. Dadurch liegt die gesamte Segmentplatte 14 und somit auch das über die Feingewindespindel 8 starr mit der Segmentplatte 14 verbundene Lagerelement 4 weiter vom zweiten Lagerelement 5 entfernt, wodurch sich der Lagerspalt 11 ergibt. Gegenüber der Figur 3a) ist die axiale Position des ersten Lagerelements 4 somit um die Höhe der erhöhten Bereiche von dem zweiten Lagerelement 5 weg gerichtet verschoben.

Um die axiale Position des Lagerelements 4, und damit den Lagerspalt 11, ausgehend von Figur 3a) definiert einzustellen, wird das Lagerelement 4 mithilfe der Feingewindespindel 8 etwas aus dem Pumpengehäuse, und damit von dem zweiten Lagerelement 5 weg gerichtet, herausgezogen. Anschließend wird die Feingewindespindel 8 um 90° gedreht, sodass die erhöhten Bereiche der Segmente 14a an der Gehäusebodenfläche 2b anliegen. Diese Position der Feingewindespindel 8 innerhalb der Segmentplatte 14 wird dann mittels der Kontermutter 9 festgehalten. Um die Segmentplatte 14 und damit die gesamte Stelleinrichtung und das erste Lagerelement 4 relativ im Gehäuseboden 2 zu fixieren, können Klemmschrauben (hier nicht gezeigt) an einer von der Rotationsachse 6 abgewandten Außenseite des Gehäusebodens 2 auf der Gehäusebodenfläche 2b in den Gehäuseboden 2 eingeschraubt sein, die eine Außenseite der Segmente 14a) und/oder 14b) zwischen sich und der Gehäusebodenfläche 2b einklemmen. Die Höhe der erhöhten Bereiche beträgt vorzugsweise 10 µm, besonders bevorzugt 8 µm, sodass eine Drehung der Segmentplatte um 90° eine Wegdifferenz des ersten Lagerelements 4 um vorzugsweise 10 µm, und entsprechend besonders bevorzugt 8 µm ergibt.

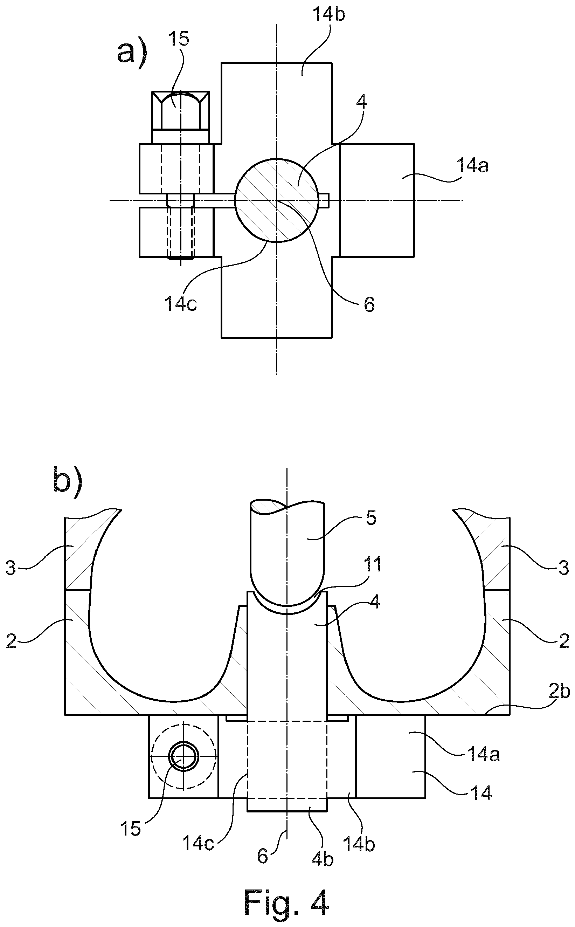

Figur 4 zeigt eine Lagerbaugruppe 1 gemäß einem vierten Ausführungsbeispiel der Erfindung sowie deren Einbau in eine Rotationsfluidpumpe in einer axialen Schnittansicht. Das Ausführungsbeispiel der Figur 4 unterscheidet sich im Wesentlichen von den Ausführungsbeispielen eins bis drei durch die Ausgestaltung der Stelleinrichtung. In Figur 4 weist das erste Lagerelement 4 einen vom zweiten Lagerelement 5 weg gerichtet axial verlängerten Schaft 4b auf. Dieser Schaft 4b greift durch eine ähnlich wie in Figur 3 ausgebildete Segmentplatte 14. Die Segmentplatte 14 liegt formschlüssig an der Gehäusebodenfläche 2b an und weist ebenfalls vier kreuzförmig angeordnete Segmente 14a, 14b auf, wobei zwei gegenüberliegende Segmente 14a wie in Figur 3 erhöhte Bereiche aufweisen. Figur 4a) zeigt eine Draufsicht auf die Segmentplatte 14. Zur Fixierung der Segmentplatte 14 am Schaft 4b weist die Segmentplatte 14 in einem der Segmente 14a eine Klemmschraube 15 auf, mittels welcher ein Durchmesser einer Durchtrittsöffnung 14c der Segmentplatte 14, durch welche der Schaft 4b hindurch tritt, einstellbar ist.

Zur Einstellung einer definierten axialen Position des ersten Lagerelements 4, und damit im Wesentlichen eines definierten Lagerspalts 11, wird die Segmentplatte 14 zunächst so gegen die Gehäusebodenfläche 2a geschoben, dass die erhöhten Bereiche der Segmente 14a in vertiefte Bereiche der Gehäusebodenfläche 2b (hier nicht gezeigt) eingreifen. Anschließend wird die relative Position zwischen dem Schaft 4b und der Segmentplatte 14 mittels Anziehen der Klemmschraube 15 fixiert. Danach wird die Segmentplatte um 90° gedreht, sodass die erhöhten Bereiche der Segmente 14a nicht mehr in die vertieften Bereiche der Gehäusebodenfläche 2b eingreifen, sondern direkt an der Gehäusebodenfläche 2b anliegen. Dadurch liegt das erste Lagerelement 4 um die Höhe der erhöhten Bereiche (vorzugsweise einschließlich 8 bis 10 µm) von dem zweiten Lagerelement 5 in Richtung des Gehäusebodens 2 entfernt, wodurch sich ein Lagerspalt 11 von etwa der Höhe der erhöhten Bereiche ergibt. Diese Stellung mit Lagerspalt 11 ist in Figur 4b) dargestellt.

Um die gesamte Stelleinrichtung und das erste Lagerelement am Gehäuseboden 2 zu befestigen, können wie im dritten Ausführungsbeispiel beschrieben Klemmschrauben auf der Gehäusebodenfläche 2b in den Gehäuseboden 2 eingeschraubt sein, welche die Außenseite der Segmentplatte mit dem Gehäuseboden 2 verklemmen.

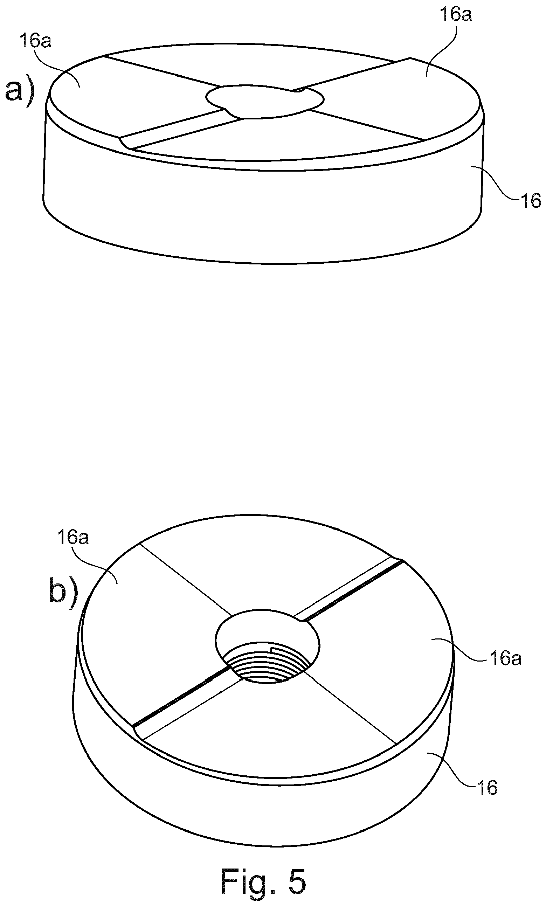

Figur 5 zeigt eine Keilscheibe 16 als Bauteil einer Lagerbaugruppe 1 gemäß einem fünften Ausführungsbeispiel der Erfindung in Perspektivansichten a) und b). Die Keilscheibe 16 ist als Kreisscheibe ausgebildet und weist auf einer kreisförmigen Oberfläche diametral angeordnete Keilflächen 16a auf, die über etwa ein Kreissegment von 45° in axialer Richtung ansteigen und aus der kreisförmigen Oberfläche herausstehen. Die Keilscheibe 16 kann anstelle der Segmentplatte 14 im dritten Ausführungsbeispiel der Figur 3 eingesetzt werden. Die erhabenen Keilflächen 16a ersetzen dabei die erhöhten Bereiche der Segmente 14a. Die vertieften Bereiche in der Gehäusebodenfläche 2b sind dann ebenfalls als entsprechend vertiefte Keilflächen ausgebildet, sodass die Keilflächen 16a zunächst in die vertieften Keilflächen der Gehäusebodenfläche 2b eingreifen können. In dieser Position ist dann der Lagerspalt 11 Null. Durch drehen der Keilscheibe um 90° drehen sich die erhöhten Keilflächen 16a vollständig aus den vertieften Keilflächen der Gehäusebodenfläche 2b heraus und liegen an der Gehäusebodenfläche 2b an. Dadurch ergibt sich ein Lagerspalt 11, der einer Maximalhöhe der Keilflächen entspricht.

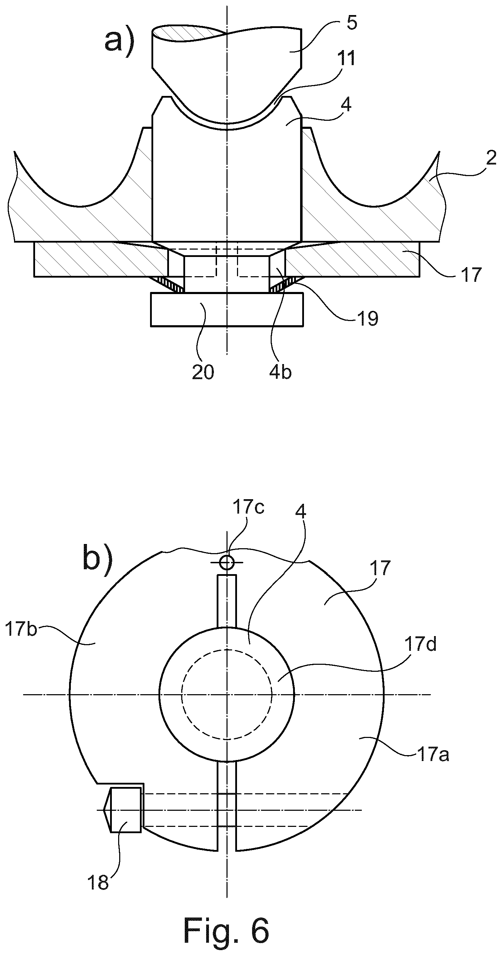

Figur 6 zeigt eine Lagerbaugruppe gemäß einem sechsten Ausführungsbeispiel der Erfindung sowie deren Einbau in eine Rotationsfluidpumpe in einer axialen Schnittansicht. In dem sechsten Ausführungsbeispiel ist an der Gehäusebodenfläche 2b eine zweiteilige Keilscheibe 17 angeordnet, die zwei über ein Gelenk 17c miteinander verbundene Halbscheiben 17a und 17b aufweist (siehe Figur 6 b)). Auf einer dem Gelenk 17c gegenüberliegenden Seite der Keilscheibe 17 ist eine Klemmschraube 18 angeordnet, mittels der im Wesentlichen ein Abstand der Halbscheiben 17a und 17b zueinander einstellbar ist. Mittig, koaxial mit der Rotationsachse 6, weist die Keilscheibe eine Durchgangsöffnung 17d auf, durch welche der Schaft 4b des ersten Lagerelements 4 hindurch greift. Ein Durchmesser der Durchgangsöffnung 17d kann ebenfalls über die Klemmschraube 18 variiert werden. Auf einer dem ersten Lagerelement 4 abgewandten Seite der Keilscheibe 17 ist der Schaft starr mit einem Abschlusselement 20 verbunden, welches ein Abrutschen der Keilscheibe 17 von dem Schaft 4b verhindert. Zwischen der Keilscheibe 17 und dem Abschlusselement 20 ist weiterhin eine Feder 19 angeordnet, welche das Abschlusselement 20 gegenüber der Keilscheibe 17 vorspannt und somit das Lagerelement 4 an die Keilscheibe 17 heranzieht. Die Durchgangsöffnung ist in axialer Richtung trichterförmig ausgestaltet. Weiterhin ist der Schaft 4b im Bereich der Durchgangsöffnung 17d keilförmig ausgebildet, wobei der Keil des Schafts 4b vorzugsweise einen flacheren Steigungswinkel aufweist als die trichterförmige Durchgangsöffnung 17d. Aufgrund der Druckkraft der Feder 19 drückt der keilförmige Schaft auf die trichterförmige Durchgangsöffnung 17d. Durch Einschrauben der Klemmschraube 18 verringert sich der Durchmesser der Durchgangsöffnung 17d, sodass die trichterförmige Durchgangsöffnung 17d auf den keilförmigen Schaft 4b drückt. Dadurch wird der keilförmige Schaft und somit das Lagerelement 4 zum Lagerelement 5 geschoben. Der Lagerspalt 11 verkleinert sich. Durch Lösen der Klemmschraube 18 vergrößert sich der Durchmesser der Durchgangsöffnung 17d, wodurch die keilförmigen Flächen des Schafts 4b und der Durchgangsöffnung weniger aufeinander drücken und die Feder 20 den Schaft 4b durch die Durchgangsöffnung 17d zieht. Der Lagerspalt 11 vergrößert sich dadurch.

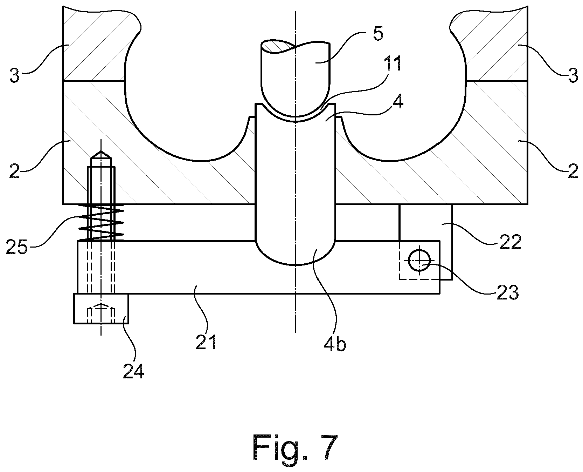

Figur 7 zeigt eine Lagerbaugruppe gemäß einem siebten Ausführungsbeispiel der Erfindung sowie deren Einbau in eine Rotationsfluidpumpe in einer axialen Schnittansicht. In dem siebten Ausführungsbeispiel wird der Schaft 4b über einen Hebel 21 in das Pumpengehäuse und somit zum zweiten Lagerelement 5 geschoben, wodurch sich der Lagerspalt 11 verkleinert, oder vom zweiten Lagerelement 5 weg aus dem Pumpengehäuse herausgezogen, sodass sich der Lagerspalt 11 vergrößert. Der Hebel 21 ist über ein Halteelement 22 und ein Gelenk 23 mit dem Gehäuseboden 2 verbunden. In einem Abstand von etwa einem Drittel der Länge des Hebels 21 zum Gelenk 23 ist der Hebel 21 mit dem Schaft 4b verbunden. Auf der dem Gelenk 23 gegenüberliegenden Seite weist der Hebel 21 eine Stellschraube 24 auf, die in den Gehäuseboden 2 einschraubbar und aus diesem herausschraubbar ist und mittels welcher ein Abstand des dem Gelenk 23 gegenüberliegen Endes des Hebels 21 von der Gehäusebodenfläche 2b veränderbar ist. Die Stellschraube 24 greift weiterhin durch eine zwischen dem Hebel 21 und der Gehäusebodenfläche 2b angeordnete Spiralfeder 25, welche den Hebel 21 gegenüber dem Gehäuseboden 2 vorspannt und somit das Gewindespiel der Stellschraube 24 verringert. Durch Ein- oder Ausschrauben der Stellschraube 24 kann eine axiale Position des ersten Lagerelements 4 in Richtung des zweiten Lagerelements 5 oder entgegen der Richtung des zweiten Lagerelements 5 geändert werden und somit der Lagerspalt 11 verkleinert oder vergrößert werden.

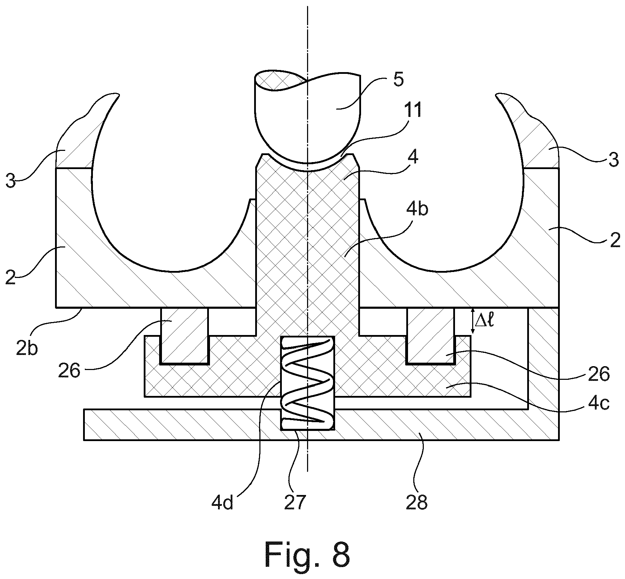

Figur 8 zeigt eine Lagerbaugruppe gemäß einem achten Ausführungsbeispiel der Erfindung sowie deren Einbau in eine Rotationsfluidpumpe in einer axialen Schnittansicht. In dem achten Ausführungsbeispiel erfolgt die Einstellung der axialen Position des ersten Lagerelements 4 bzw. des Lagerspaltes 11 über Elemente 26 mit einem vorbestimmten Wärmeausdehnungskoeffizienten. Hierzu ist der Schaft 4b starr mit einer Platte 4c verbunden, die parallel zur Gehäusebodenfläche 2b und beabstandet zum Gehäuseboden 2 verläuft. Zwischen dem Gehäuseboden 2 und der Platte 4c sind die Elemente 26 mit vorbestimmtem Wärmeausdehnungskoeffizient angeordnet. Auf einer der Gehäusebodenfläche 2b abgewandten Seite der Platte 4c ist eine parallel und beabstandet zur Platte 4c verlaufende und starr mit dem Gehäuseboden 2 verbundene Abstützplatte 28 angeordnet. Zwischen der Platte 4c und der Abstützplatte 28 ist weiterhin eine Spiralfeder 27 angeordnet, die eine gegenseitige Druckkraft auf die Platte 4c und die Abstützplatte 28 ausübt und dadurch die Platte 4c gegenüber der Abstützplatte 28 vorspannt. Zur Führung der Spiralfeder 27 ist diese in gegenüberliegende zylinderförmige Nuten 4d und 28a in der Platte 4c bzw. der Abstützplatte 28 im Bereich der Rotationsachse 6 eingesetzt.

Die Einstellung der axialen Position des ersten Lagerelements erfolgt dadurch, dass die Elemente 26 eine vorbestimmte Temperaturdifferenz erfahren und sich daraufhin in definiertem Maße Ausdehnen oder Zusammenziehen. Im Falle einer Verkleinerung des Lagerspalts 11 wird die Temperatur der Elemente 26 erniedrigt, woraufhin diese einen verringerten Druck auf die Platte 4c ausüben. Überwindet dann die Feder 27 die Druckkraft der Elemente 27 wird das erste Lagerelement 4 zum zweiten Lagerelement 5 geschoben. Im Falle einer Verkleinerung des Lagerspalts 11 wird die Temperatur der Elemente 26 erhöht, wodurch sich die Elemente ausdehnen und sich ihre Druckkraft auf die Platte 4c erhöht. Überwindet die Druckkraft der Elemente 26 die Druckkraft der Feder 27 wird das erste Lagerelement 4 von dem zweiten Lagerelement 5 weggezogen.

Für die Elemente 26 werden vorzugsweise Metalle mit einer großen Wärmeausdehnung verwendet. Beispielsweise ist eine 20-fache Wärmeausdehnung notwendig, um den Lagerspalt 11 einzustellen.

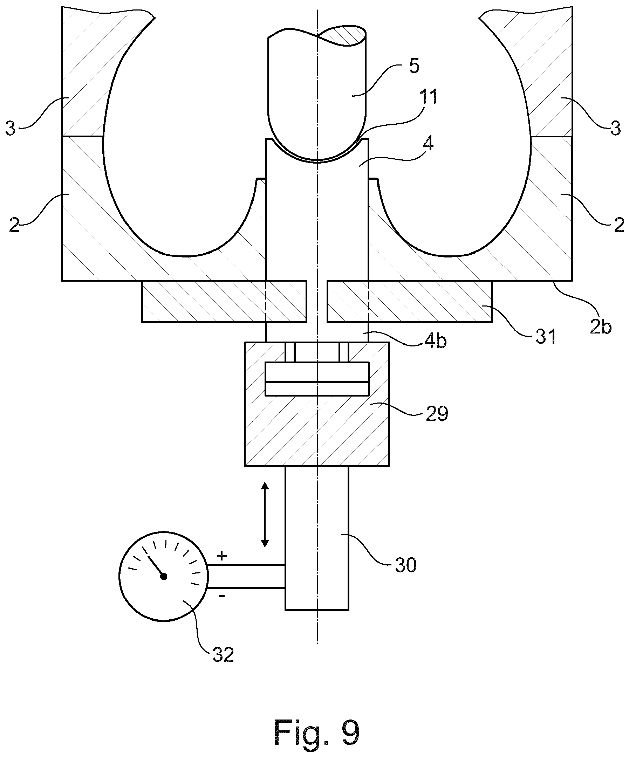

Figur 9 zeigt eine Lagerbaugruppe gemäß einem neunten Ausführungsbeispiel der Erfindung sowie deren Einbau in eine Rotationsfluidpumpe in einer axialen Schnittansicht. Bei diesem neunten Ausführungsbeispiel ist der Schaft 4b über eine Kupplung 29 starr mit einem Piezoaktuator 30 verbunden. Mittels der mit dem Piezoaktuator 30 verbundenen Regelungseinrichtung 32 kann eine definierte Spannung an den Piezoaktuator 30 angelegt werden. Dieser dehnt sich aufgrund der Spannungsänderung aus oder zieht sich zusammen und kann dadurch über den Schaft 4b die axiale Position des ersten Lagerelements 4 variieren. Ist die axiale Position des ersten Lagerelements 4 bzw. der Lagerspalt 11 eingestellt, kann die axiale Position des Lagerelements 4 über ein an der Gehäusebodenfläche 2b anliegendes Klemmelement 31 fixiert werden.

Zur Einstellung der axialen Position bzw. des Lagerspalts wird die gesamte Rotationsfluidpumpe in eine Haltevorrichtung eingesetzt. Die Kupplung 29 wird vorzugsweise spielfrei mit dem Schaft 4b verbunden und kann nach Einstellung der axialen Position des ersten Lagerelements 4 bzw. des Lagerspalts von dem Schaft 4b wieder gelöst werden. Insbesondere das hier beschriebene neunte Ausführungsbeispiel ermöglicht eine besonders genaue und reproduzierbare Einstellbarkeit der axialen Position des ersten Lagerelements 4.

## Patentansprüche

1. Lagerbaugruppe zum Lagern eines um eine Rotationsachse rotierbaren Rotors, insbesondere für eine Rotationsfluidpumpe oder Rotationsblutpumpe, umfassend:
einen Grundkörper,
ein relativ zum Grundkörper in Richtung der Rotationsachse verschiebbares Lagerelement zum Aufnehmen des Rotors, und
eine mit dem Lagerelement verbundene Stelleinrichtung zum Verschieben des Lagerelements in der Richtung der Rotationsachse um einen vorbestimmten Weg, wobei der vorbestimmte Weg ≤ 500 µm beträgt.

2. Lagerbaugruppe nach dem vorhergehenden Anspruch, **dadurch gekennzeichnet, dass** der vorbestimmte Weg ≤ 100 µm, insbesondere ≤ 50 µm, bevorzugt ≤ 20 µm, besonders bevorzugt ≤ 10 µm, insbesondere ≤ 5 µm beträgt.

3. Lagerbaugruppe nach einem der vorhergehenden Ansprüche, **dadurch gekennzeichnet, dass** die Stelleinrichtung ein Schraubelement umfasst, welches derart mit dem Lagerelement verbunden ist, dass eine Drehung des Schraubelements um einen vorbestimmten Winkel eine Verschiebung des Lagerelements um den vorbestimmten Weg bewirkt.

4. Lagerbaugruppe nach dem vorhergehenden Anspruch, **dadurch gekennzeichnet, dass** eine Drehung des Schraubelements um 45 ° eine Verschiebung des Lagerelements um 25 µm bewirkt.

5. Lagerbaugruppe nach einem der vorhergehenden Ansprüche, **dadurch gekennzeichnet, dass** die Stelleinrichtung eine Feingewindespindel und eine Gewindescheibe umfasst, wobei die Gewindescheibe mit dem Grundelement starr verbunden ist, und wobei die Feingewindespindel in der Richtung der Rotationsachse mit dem Lagerelement starr verbunden und in die Gewindescheibe eingeschraubt ist, sodass eine Schraubbewegung der Feingewindespindel in der Gewindescheibe um einen vorbestimmten Winkel eine Verschiebung des Lagerelements um den vorbestimmten Weg bewirkt.

6. Lagerbaugruppe nach dem vorhergehenden Anspruch, **dadurch gekennzeichnet, dass** zwischen dem Lagerelement und der Gewindescheibe ein Federelement angeordnet ist, welches die Feingewindespindel gegenüber der Gewindescheibe vorspannt.

7. Lagerbaugruppe nach einem der Ansprüche 1 bis 4, **dadurch gekennzeichnet, dass** die Stelleinrichtung eine Feingewindespindel, eine Segmentplatte mit mindestens vier kreuzförmig angeordneten Segmenten und eine Kontermutter aufweist, wobei die Feingewindespindel in der Richtung der Rotationsachse starr mit dem Lagerelement verbunden ist und auf einer dem Lagerelement abgewandten Seite in die Segmentplatte eingeschraubt und mittels der Kontermutter in der Segmentplatte fixiert ist, und wobei die Segmentplatte in zwei gegenüberliegenden Segmenten zum Grundkörper hin erhöhte Bereiche mit einer vorbestimmten Höhe aufweist, und der Grundkörper den erhöhten Bereichen der Segmentplatte gegenüberliegende vertiefte Bereiche aufweist, sodass in einer ersten Position der Segmentplatte die erhöhten und vertieften Bereiche ineinander greifen können, und sodass ein Drehen der Segmentplatte in eine zweite Position, in welcher die erhöhten und vertieften Bereiche nicht ineinander greifen, eine Verschiebung des Lagerelements um den vorbestimmten Weg bewirkt, wobei der vorbestimmte Weg der vorbestimmten Höhe der erhöhten Bereiche entspricht.

8. Lagerbaugruppe nach einem der Ansprüche 1 bis 4, **dadurch gekennzeichnet, dass** die Stelleinrichtung eine Segmentplatte mit mindestens vier kreuzförmig angeordneten Segmenten und einer Klemmeinrichtung aufweist, wobei das Lagerelement in der Richtung der Rotationsachse in die Segmentplatte eingesteckt und mittels der Klemmeinrichtung fixiert ist, und wobei die Segmentplatte in zwei gegenüberliegenden Segmenten zum Grundkörper hin erhöhte Bereiche mit einer vorbestimmten Höhe aufweist, und der Grundkörper den erhöhten Bereichen der Segmentplatte gegenüberliegende vertiefte Bereiche aufweist, sodass in einer ersten Position der Segmentplatte die erhöhten und vertieften Bereiche ineinandergreifen können, und sodass ein Drehen der Segmentplatte in eine zweite Position, in welcher die erhöhten und vertieften Bereiche nicht ineinander greifen, eine Verschiebung des Lagerelements um den vorbestimmten Weg bewirkt, wobei der vorbestimmte Weg der vorbestimmten Höhe der erhöhten Bereiche entspricht.

9. Lagerbaugruppe nach einem der zwei vorhergehenden Ansprüche, **dadurch gekennzeichnet, dass** die erhöhten Bereiche mittels eines Spanprozesses, insbesondere mittels eines Schleifprozesses, und/oder mittels eines additiven Prozesses, insbesondere mittels eines Aufdampfprozesses, eines Druckprozesses und/oder eines Beschichtungsprozesses, herstellbar sind.

10. Lagerbaugruppe nach einem der Ansprüche 1 bis 4, **dadurch gekennzeichnet, dass** die Stelleinrichtung eine Keilscheibe mit zwei diametral versetzt angeordneten, in einer Umfangsrichtung der Keilscheibe geneigten Keilflächen umfasst, wobei das Lagerelement in die Keilscheibe eingeschraubt ist, sodass ein Drehen der Keilscheibe um einen vorbestimmten Winkel eine Verschiebung des Lagerelements um den vorbestimmten Weg bewirkt.

11. Lagerbaugruppe nach einem der Ansprüche 1 bis 4, **dadurch gekennzeichnet, dass** das Lagerelement auf einer dem aufzunehmenden Rotor abgewandten Seite des Lagerelements einen keilförmigen Abschnitt aufweist, und die Stelleinrichtung ein scheibenförmiges Element mit einer trichterförmigen Durchgangsöffnung aufweist, wobei das Lagerelement mit dem keilförmigen Abschnitt in die trichterförmige Durchgangsöffnung eingesteckt ist und starr mit einem Abschlusselement verbunden ist, und wobei weiterhin die Stelleinrichtung ein Federelement, welches zwischen dem scheibenförmigen Element und dem Abschlusselement angeordnet ist und das scheibenförmige Element gegenüber dem Lagerelement vorspannt, und eine in dem scheibenförmigen Element angeordnete Klemmschraube umfasst, mit welcher ein Durchmesser der keilförmigen Durchgangsöffnung um einen vorbestimmten Betrag veränderbar ist, sodass ein Verändern des Durchmessers um einen vorbestimmten Differenzdurchmesser eine Verschiebung des Lagerelements um den vorbestimmten Weg bewirkt.

12. Lagerbaugruppe nach einem der Ansprüche 1 bis 4, **dadurch gekennzeichnet, dass** die Stelleinrichtung einen Hebel umfasst, der mit dem Lagerelement verbunden ist, sodass eine Veränderung der Neigung des Hebels relativ zum Grundkörper um einen vorbestimmten Differenzwinkel eine Verschiebung des Lagerelements um den vorbestimmten Weg bewirkt.

13. Lagerbaugruppe nach einem der Ansprüche 1 bis 4, **dadurch gekennzeichnet, dass** die Stelleinrichtung ein Element mit einem vorbestimmten Wärmeausdehnungskoeffizienten umfasst, wobei das Element derart mit dem Lagerelement verbunden ist, dass eine Änderung einer Temperatur des Elements um einen vorbestimmten Temperaturbetrag eine Verschiebung des Lagerelements um den vorbestimmten Weg bewirkt.

14. Lagerbaugruppe nach einem der Ansprüche 1 bis 4, **dadurch gekennzeichnet, dass** die Stelleinrichtung ein Piezo-Element umfasst, welches mit dem Lagerelement derart verbunden ist, insbesondere lösbar derart verbunden ist, dass ein Anlegen einer vorbestimmten elektrischen Spannung an das Piezo-Element ein Verschieben des Lagerelements um den vorbestimmten Weg bewirkt.

15. Rotationsfluidpumpe zum Fördern eines Fluids, insbesondere Rotationsblutpumpe zum Fördern von Blut, umfassend eine Lagerbaugruppe nach einem der vorhergehenden Ansprüche.
